# EUROPEAN PATENT SPECIFICATION

(11) **EP 1 747 781 B1**
(45) Date of publication and mention of the grant of the patent: **17.02.2010**
(21) Application number: 05748549.2
(22) Date of filing: 13.05.2005
(51) Int. Cl.: A61K 31/665, A61K 47/10, A61P 31/04, A61P 13/02

(54) **STABLE ORAL PHARMACEUTICAL PREPARATION COMPRISING FOSFOMYCIN, WHICH IS INTENDED FOR DIABETIC PATIENTS**
STABILE ORALE PHARMAZEUTISCHE ZUBEREITUNG AUS FOSFOMYCIN FÜR DIABETIKER
PREPARATION PHARMACEUTIQUE ORALE DE FOSFOMYCINE STABLE ET DESTINEE A DES DIABETIQUES

(30) Priority: 18.05.2004 ES 200401190
(43) Date of publication of application: 31.01.2007
(73) Proprietor: Simbec Iberica, SL, 08007 Barcelona (ES)
(72) Inventor: SUÑE NEGRE, José M., E-08028 Barcelona (ES); TICÓ GRAU, José Ramon, E-08034 Barcelona (ES); MESTRE CASTELL, José, 08960 SANT JUST DESVERN (BARCELONA) (ES)
(74) Representative: Gislon, Gabriele
(86) International application number: PCT/ES2005/000264
(87) International publication number: WO 2005/110427

(56) References cited:
- ES-A1- 2 103 682
- ES-A1- 8 200 700
- ES-A6- 2 020 790
- US-A1- 2004 022 866

## Description

### Technical field

The present invention relates to an oral pharmaceutical preparation in powder or granule form for a solution containing fosfomycin as an active principle, which is suitable for diabetic patients and is stable as a finished product.

### Prior art

Fosfomycin is an antibiotic with a bactericidal action which acts by inhibiting the synthesis of the bacterial wall. It is a wide-spectrum antibiotic although it acts more intensely on gram-positive bacteria.

Fosfomycin trometamol is the international nonproprietary name (INN) corresponding to the compound with the chemical name mono(2-ammonium-2-hydroxymethyl-1,3-propanediol) (2R-cis)-1,2-epoxypropylphosphonate, the formula of which is the following:

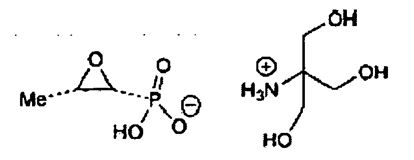

Fosfomycin trometamol is an antibiotic indicated in prophylaxis and the treatment of uncomplicated acute infections of the lower urinary tract.

Spanish patent application ES495870 describes a preparation in sachets for the oral administration of fosfomycin trometamol containing sodium carboxymethylcellulose, lactose, titanium dioxide, sucrose and a flavoring as excipients. Sucrose is the major component, forming between 56% and 75% by weight of the formulation.

Spanish patent application ES2020790 describes a water-soluble granulated fosfomycin trometamol formulation containing saccharin, sucrose and flavorings as excipients. Sucrose represents 27% of the formulation.

In these formulations described in the state of the art, the presence of a component with aldehyde groups (sucrose) and a component with primary amino groups (trometamol) is identified in the same solid formulation.

The simultaneous presence of aldehyde groups and primary amino groups poses a high risk of chemical interaction, according to the known Maillard reaction, due to the fact that aldehyde groups (like those in sucrose) interact with primary amino groups (like those in trometamol) generally causing undesirable colour formation in the formulation.

Furthermore, the presence of sucrose in the formulations of the state of the art does not allow the administration thereof to persons suffering from diabetes.

There is therefore the need to develop new oral fosfomycin preparations with an excipient system that is compatible with the active principle which do not pose any risk of undesirable colour formation and which are suitable for administration to diabetic patients.

The US patent application No. 2004/022866 discloses pharmaceutical compositions of fosfomycin tromethamol stabilized by the addition of a stabilizer such as sodium citrate. The exemplified compositions contain as sweeteners: aspartame, sucrose, fructose, sodium saccharin and sorbitol. The Merck Index shows that one of the uses of mannitol is as excipient and diluent in pharmacy (The Merck Index, 13 edition, page 5767)

The authors of the present invention have developed a fosfomycin preparation preventing the risk of chemical interaction and which can further be administered to diabetic patients.

### Object of the invention

The object of the present invention is an oral pharmaceutical preparation in powder or granule form for a solution containing fosfomycin as an active principle, which is suitable for diabetic patients and is stable as a finished product.

### Detailed description of the invention

The pharmaceutical preparation comprising fosfomycin trometamol for oral administration in powder or granule form for a solution object of the invention is **characterized in that** it contains as excipients:
- xylitol.
- an artificial sweetening agent which is selected from the group including: acesulfame, aspartame, saccharin, alitame and/or cyclamate, and
is substantially sucrose-free.

The pharmaceutical preparation object of the invention can further comprise other optional excipients such as for example, flavoring and aromatic agents.

### Active principle

The pharmaceutical preparation object of the invention comprises fosfomycin trometamol as the active principle. As mentioned above, fosfomycin trometamol is the salt of fosfomycin with trometamol.

The fosfomycin trometamol content in the preparation object of the invention can be comprised between 60 and 80% by weight over the total weight of the formulation, preferably between 65 and 75%.

The pharmaceutical preparation object of the invention can be presented in a single-dose form for its dissolution which can contain between 1 and 4 g of fosfomycin expressed as free acid, corresponding to between 1.9 and 7.5 g respectively of fosfomycin trometamol.

The single-dose sachets preferably contain between 2 and 3 g of fosfomycin expressed as free acid, corresponding to between 3.8 and 5.6 g respectively of fosfomycin trometamol.

### Mannitol and/or xylitol excipients

The preparations object of the invention are **characterized in that** they are essentially sucrose-free. By substantially sucrose-free it is understood that it is either a preparation that does not contain sucrose or a preparation containing a small amount of sucrose that does not prevent its administration to diabetic persons and/or does not pose the risk of undesirable colour formation therein.

The preparations of the invention comprise xylitol or mannitol and xylitol as main excipients, which advantageously substitute the sucrose of the preparations of the prior art, providing the preparation with the suitable volume without showing the previously indicated drawbacks of sucrose.

Mannitol and xylitol are polyhydric alcohols, therefore since they do not have aldehyde groups, they do not pose the risk of interaction with the active principle, preventing the possible undesirable colour formation.

In addition to providing a sweet taste to the preparation, said excipients have the advantage of not being absorbed in the gastrointestinal tract, therefore they can be administered to diabetic persons.

Furthermore, mannitol and xylitol are less hygroscopic than sucrose, which is an important advantage in manufacturing processes.

The amount of mannitol and xylitol present in the preparation can be easily determined conventionally by a person skilled in the art, in order to obtain a product in powder or granule form with good fluidity properties and further having a pleasant taste.

The mannitol and xylitol content can generally be comprised between 20 and 40% by weight over the total weight of the formulation, preferably between 25 and 35%.

In a preferred embodiment, the pharmaceutical preparation object of the invention comprises a mixture of mannitol and xylitol.

More preferably, the ratio between mannitol and xylitol is comprised respectively between 70:30 and 30:70 by weight.

The ratio 50:50 by weight between mannitol and xylitol is especially preferred.

### Artificial sweetener

The pharmaceutical preparation object of the invention contains an artificial sweetener to reinforce the sweetening power of the mannitol and/or xylitol present therein.

The artificial sweetener is selected from the group including: acesulfame, aspartame, saccharin, alitame and/or cyclamate. The preparation preferably comprises aspartame.

Aspartame is a sweetening substance which is effective in low proportions because its sweetening power is 180 to 200 times greater than sucrose and it further offers the same sweet taste as sucrose. Furthermore, since aspartame does not contain aldehyde groups, it is a substance that is compatible with trometamol.

The amount of aspartame present in the preparation is adjusted by means of routine work to obtain a pleasant taste. Generally, the aspartame content present in the preparation can be comprised between 0.1 and 1% by weight over the total weight of the formulation.

In another preferred embodiment, the artificial sweetener comprises a combination of aspartame and saccharin.

Saccharin is a sweetening agent which has a sweetening power that is approximately 500 times greater than that of sucrose but it has residual metallic taste that is perceived by 25% of the population. Therefore, saccharin is generally used in combination with other sweeteners, such that upon reducing its content in the formulation the residual taste is also minimized.

The use of sodium saccharin as a part of the sweetening system would be contraindicated especially in patients with cardiovascular pathologies.

To that end, a mixture of acid saccharin and free trometamol is preferably used in the pharmaceutical preparation object of the invention in order to form the salt *in situ* at the time of dissolving the preparation for its administration.

The pharmaceutical preparation object of the invention is set forth as a product in powder or granule form for an oral solution which can be obtained by pharmaceutical technology processes that are well known by persons skilled in the art. The following can be mentioned among said processes: the mixing of all the homogenized components in a mixing drum to obtain a product in powder form, and the mixing and kneading with a short chain alcohol, for example absolute ethanol or isopropanol, followed by granulation to obtain a granulated product.

The pharmaceutical preparation can further contain flavoring and aromatic agents in suitable concentrations, for example, orange, tangerine, raspberry, strawberry, coconut flavorings, etc.

Once the pharmaceutical preparation has been obtained in powder or granule form for a solution, the latter can be dosed in single-dose sachets with a fosfomycin content, expressed as free acid, comprised between 1 and 4 g, as indicated previously.

The obtained pharmaceutical preparation object of the invention has good stability, fluidity and water-solubility properties.

The pharmaceutical preparation object of the invention has an excipient system that is absolutely compatible with the active principle which confers a good stability as a finished product thereto.

Thus, when a commercial preparation containing fosfomycin trometamol and sucrose is dissolved in water at 55°C, after 45 days said solution has a dark yellow colour that is characteristic of the Maillard reaction, whereas in the same conditions the solution of the preparation of the invention remains colourless.

The pharmaceutical preparation object of the invention is intended for oral administration by means of its dissolution in water or any other aqueous liquid.

The recommended dose is dissolved in a sufficient amount of water or any other aqueous beverage, for example, half a glass, and it is administered immediately after its preparation.

The following examples are set forth for the purpose of providing the person skilled in the art with a detailed explanation of specific embodiments within the invention.

### Example 1 Obtaining the pharmaceutical preparation by simple mixing

The components forming the pharmaceutical preparation are sieved separately through a sieve with a 0.8 mm mesh opening, for the purpose of homogenizing them.

105.08 g of trometamol fosfomycin, corresponding to 56 g of free fosfomycin, 20.53 g of mannitol, 20.53 of xylitol, 0.75 g of aspartame, 1.31 g of orange flavoring and 1.31 g of tangerine flavoring are mixed in the mixing drum.

The group of components is homogenized in the mixing drum for 20 minutes.

The powdery solid is dosed in sachets at 5.34 g or 8.01 g per sachet, corresponding respectively to 2 g or 3 g of free fosfomycin per sachet.

### Example 2 Obtaining the pharmaceutical preparation by kneading with ethanol and subsequent granulation

105.08 g of trometamol fosfomycin, 0.75 g of aspartame, 20.53 g of mannitol and 20.53 of xylitol are weighed and sieved separately through a sieve with a 0.8 mm mesh opening, for the purpose of homogenizing them.

Said components are then mixed in a biconical mixer for 5 minutes and a solution of 0.30 g of acid saccharin and 0.21 g of trometamol in 75 ml of absolute ethanol is then added to said mixture.

A thick paste is obtained which is subjected a granulation process.

The granules are dried in an oven.

Finally, 1.31 g of tangerine flavoring and 1.31 g of orange flavoring are added to the granules in a solid mixer.

It is mixed for 5 minutes to obtain the absorption of the liquid flavorings in the granules.

The obtained powdery solid is dosed in sachets at 5.359 g or 8.039 g per sachet, corresponding respectively to 2 g or to 3 g of fosfomycin per sachet.

## Claims

1. A pharmaceutical preparation comprising fosfomycin trometamol for oral administration in powder or granule form for a solution **characterized in that** it comprises as excipients:
- xylitol,
- an artificial sweetening agent which is selected from the group including: acesulfame, aspartame, saccharin, alitame and/or cyclamate,
and is substantially sucrose-free.

2. A preparation according to claim 1, **characterized in that** the fosfomycin trometamol content is comprised between 60 and 80% by weight over the total weight of the formulation.

3. A preparation according to claim 2, **characterized in that** the fosfomycin trometamol content is comprised between 65 and 75% by weight over the total weight of the formulation.

4. A preparation according to any of claims 1 to 3, **characterized in that** it comprises a mixture of mannitol and xylitol.

5. A preparation according to claim 4, **characterized in that** the mannitol and xylitol content is comprised between 20 and 40% by weight over the total weight of the formulation.

6. A preparation according to claim 5, **characterized in that** the mannitol and xylitol content is comprised between 25 and 35% by weight over the total weight of the formulation.

7. A preparation according to claim 6, **characterized in that** the ratio between mannitol and xylitol is respectively comprised between 70:30 and 30:70 by weight.

8. A preparation according to claim 7, **characterized in that** the ratio between mannitol and xylitol is 50:50 by weight.

9. A preparation according to claims 1 to 8, **characterized in that** it comprises aspartame as an artificial sweetener.

10. A preparation according to claim 9, **characterized in that** the artificial sweetener comprises a mixture of aspartame and saccharin.

## Patentansprüche

1. Pharmazeutische Zubereitung umfassend Fosfomycin-Trometamol zur oralen Verabreichung in Pulver- oder Granulatform für eine Lösung, **dadurch gekennzeichnet, dass** sie als Arzneiträger Folgendes umfasst:
- Xylitol,
- ein künstliches Süßungsmittel, welches aus der Gruppe ausgewählt wird, die Folgendes beinhaltet: Acesulfam, Aspartam, Saccharin, Alitam und/oder Cyclamat,
und im Wesentlichen sucrosefrei ist.

2. Zubereitung nach Anspruch 1, **dadurch gekennzeichnet, dass** der Fosfomycin-Trometamol-Gehalt zwischen 60 und 80 Gewichts-% in Bezug auf das Gesamtgewicht der Rezeptur liegt.

3. Zubereitung nach Anspruch 2, **dadurch gekennzeichnet, dass** der Fosfomycin-Trometamol-Gehalt zwischen 65 und 75 Gewichts-% in Bezug auf das Gesamtgewicht der Rezeptur liegt.

4. Zubereitung nach einem der Ansprüche 1 bis 3, **dadurch gekennzeichnet, dass** sie eine Mischung aus Mannitol und Xylitol umfasst.

5. Zubereitung nach Anspruch 4, **dadurch gekennzeichnet, dass** der Mannitol- und Xylitol-Gehalt zwischen 20 und 40 Gewichts-% in Bezug auf das Gesamtgewicht der Rezeptur liegt.

6. Zubereitung nach Anspruch 5, **dadurch gekennzeichnet, dass** der Mannitol- und Xylitol-Gehalt zwischen 25 und 35 Gewichts-% in Bezug auf das Gesamtgewicht der Rezeptur liegt.

7. Zubereitung nach Anspruch 6, **dadurch gekennzeichnet, dass** das Gewichtsverhältnis zwischen Mannitol und Xylitol zwischen jeweils 70:30 und 30:70 liegt.

8. Zubereitung nach Anspruch 7, **dadurch gekennzeichnet, dass** das Gewichtsverhältnis zwischen Mannitol und Xylitol 50:50 beträgt.

9. Zubereitung nach den Ansprüchen 1 bis 8, **dadurch gekennzeichnet, dass** sie Aspartam als künstlichen Süßstoff umfasst.

10. Zubereitung nach Anspruch 9, **dadurch gekennzeichnet, dass** der künstliche Süßstoff eine Mischung aus Aspartam und Saccharin umfasst.

## Revendications

1. Une préparation pharmaceutique comportant du trométamol phosphomycine pour administration orale en poudre ou en granulé **caractérisée en ce qu'**elle comporte comme excipients:
- xylitol
- un agent sucrant artificiel qui est sélectionné du groupe comportant: acésulphame, aspartame, saccharine, alitame et/ou cyclamate,
et qui est sensiblement sans saccharose.

2. Une préparation conformément à la revendication 1 **caractérisée en ce que** la teneur de trométamol phosphomycine est comprise entre les 60 et 80% du poids sur le poids total de la formulation.

3. Une préparation conformément à la revendication 2, **caractérisée en ce que** la teneur de trométamol phosphomycine est comprise entre les 65 et 75% du poids sur le poids total de la formulation.

4. - Une préparation conformément à une quelconque des revendications 1 à 3, **caractérisée en ce qu'**elle comporte un mélange de mannitol et de xylitol.

5. - Une préparation conformément à la revendication 4, **caractérisée en ce que** la teneur de mannitol et de xylitol est comprise entre les 20 et 40% du poids sur le poids total de la formulation.

6. - Une préparation conformément à la revendication 5, **caractérisée en ce que** la teneur de mannitol et de xylitol est comprise entre les 25 et 35% du poids sur le poids total de la formulation.

7. - Une préparation conformément à la revendication 6, **caractérisée en ce que** le ratio entre le mannitol et le xylitol est respectivement compris entre 70:30 et 30:70 du poids.

8. - Une préparation conformément à la revendication 7, **caractérisée en ce que** le ration entre le mannitol et le xylitol est 50:50 du poids.

9. - Une préparation conformément aux revendications 1 à 8, **caractérisée en ce qu'**elle comporte de l'aspartame comme sucrant artificiel.

10. - Une préparation conformément à la revendication 9, **caractérisée en ce que** le sucrant artificiel comporte un mélange d'aspartame et de saccharine
